# DEMANDE DE BREVET EUROPEEN

(11) **EP 0 579 563 A1**
(43) Date de publication de la demande: **19.01.1994**
(21) Numéro de dépôt: 93420306.8
(22) Date de dépôt: 16.07.1993
(51) Int. Cl.: A61B 17/58, A61F 2/36

(54) **Ensemble prothétique fémoral**

(30) Priorité: 16.07.1992 FR 9209117
(71) Demandeur: MEDINOV SA, F-42312 Roanne Cedex (FR)
(72) Inventeur: Grammont, Paul, F-21000Dijon (FR); Colombier, Michel, F-42131 Saint Cyr (FR)
(74) Mandataire: Dupuis, François

(57) **Abrégé**

L'ensemble comprend un élément (1) dont la forme et le profil en section sont déterminés pour permettre son impaction au niveau de la partie métaphysaire du fémur (F), ledit élément (1), qui présente directement ou de manière rapportée une tête d'articulation (3) coopérant avec la cavité cotyloïde, étant agencé pour l'introduction d'au moins un clou centromédullaire (2).

## Description

Plus particulièrement, l'invention concerne les techniques d'enclouage et d'arthroplastie de la hanche, pour le traitement notamment des fractures perthrocantériennes ou associées.

On sait qu'il existe également des risques de fractures de la diaphyse fémorale chez les porteurs de prothèses. Le composite proximal entre l'os et la prothèse est stable et rigide et, en cas de traumatisme, résiste aux énergies traumatiques.

En outre, les diaphyses, souvent déminéralisées chez les personnes agées, sont d'autant plus fragiles au choc, que l'extrémité proximale ne l'est pas.

Les traitements utilisés ne donnent pas satisfaction et mettent en oeuvre, essentiellement deux techniques.

Selon une première technique, on utilise un enclouage sous forme d'une vis céphalique disposée au milieu du col et de la tête du fémur et susceptible de coopérer avec un clou centromédullaire ou une plaque. Un tel système est souvent instable, car l'os est de mauvaise qualité, de sorte que les plaques peuvent se démonter. De même, des fragments osseux peuvent être entrainés en rotation quand on visse la vis céphalique.

Suivant une autre technique, on utilise une prothèse comprenant une tige fémorale et une partie métaphysaire prolongée par un col latéralisé recevant directement ou d'une manière rapportée, une tête sphérique destinée à coopérer avec la cavité cotyloïde de l'os illiaque. Un tel système est stable à court terme, mais peut entraîner, à long terme, la dégénération de l'os, si la congruance n'est pas parfaite en partie proximale. Il peut y avoir un pontage des corticales proximales si la tige est bloquée distalement

L'invention s'est fixée pour but de remédier à ces inconvénients, de manière simple et sûre efficace

Le problème que se propose de résoudre l'invention est d'atteindre les trois objectifs essentiels suivants :
- solidariser le grand trochanter avec la prothèse pour reconfectionner le hauban naturel entre ces deux parties
- permettre un enclouage en cas de fracture secondaire en bout de tige
- rendre cet implant facilement extractible en cas de besoin.

Pour résoudre ce problème et atteindre ces objectifs, il a été conçu et mis au point un ensemble qui comprend un élément dont la forme et le profil en section sont déterminés pour permettre son impaction au niveau de la partie métaphysaire du fémur, ledit élément, qui présente directement ou de manière rapportée une tête d'articulation coopérant avec la cavité cotyloïde, étant agencé pour l'introduction d'au moins un clou centromédullaire.

Pour résoudre le problème posé de l'introduction du clou centromédullaire, I'élément métaphysaire présente un canal débouchant.

Dans le cas de certaines fractures, telles que spiroïdes ou pertes de substances, le clou est fixé au niveau de la partie distale et parfois au niveau de la partie proximale.

Le clou peut être déformable élastiquement en étant rectiligne, cintré ou constitué par un faisceau de plusieurs petites tiges.

Compte-tenu du problème posé de restaurer l'articulation de la hanche, I'élément métaphysaire présente un col latéralisé recevant directement de manière rapportée, une tête sphérique d'articulation coopérant avec la cavité cotyloïde.

L'invention est exposée, ci-après plus en détail à l'aide des dessins annexés, dans lesquels :

La figure 1 est une vue en coupe longitudinale de l'ensemble prothétique impacté dans le canal médullaire.

La figure 2 est une vue en coupe transversale considérée selon la ligne 2.2 de la figure 1.

La figure 3 est une vue correspondante à la figure 1, dans le cas où le clou centromédullaire est fixé au niveau de sa partie distale.

Selon l'invention, l'ensemble prothétique comprend, en combinaison, un élément (1) destiné à prendre appui au niveau de la partie métaphysaire du fémur (F) et un élément (2), sous forme d'un clou centromédullaire.

L'élément (1) présente une forme et un profil en section déterminés pour son impaction dans la partie métaphysaire du fémur. De manière connue, les faces latérales (1a) de l'élément (1) peuvent présenter tout agencement et/ou revêtement de surface pour favoriser son impaction ou son accrochage, notamment dans le cadre d'une fixation sans ciment.

Cet élément (1) présente, à la façon d'une prothèse classique de la hanche, un col latéralisé (1b) recevant directement ou d'une manière rapportée, une tête sphérique (3) coopérant avec la cavité cotyloïde de l'os illiaque. Cet élément (1) permet donc de reconstituer la fonction d'articulation entre le fémur et le bassin en constituant un appui métaphysaire.

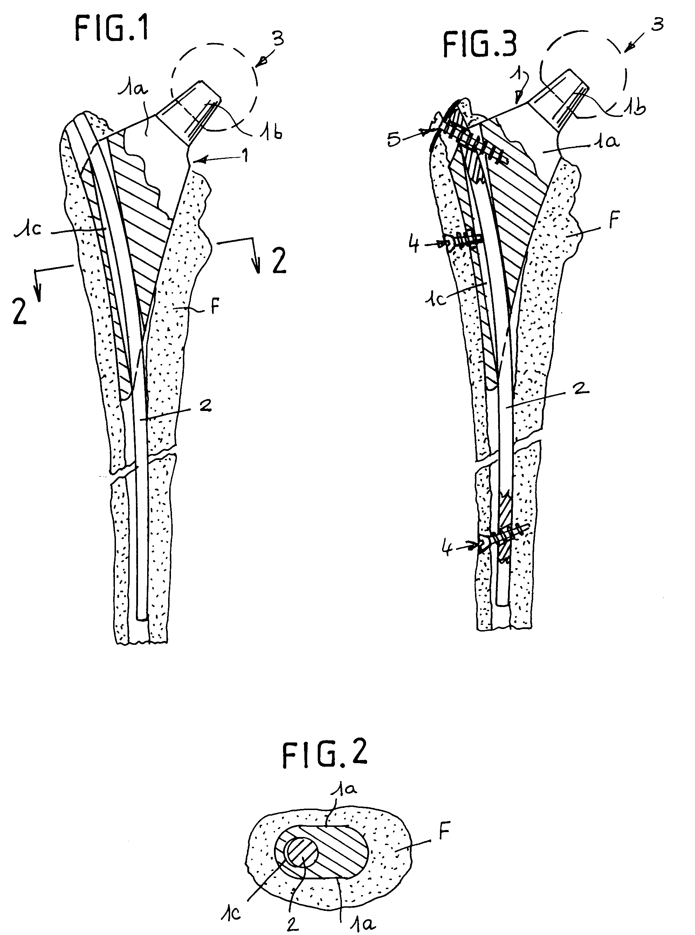

L'élément métaphysaire (1) présente un canal débouchant (1c) pour l'introduction du clou centromédullaire (2). Le clou (2) est déformable élastiquement en étant rectiligne ou cintré. Ce clou déborde très légèrement de la partie proximale de l'élément métaphysaire (1) et coopère avec le canal médullaire du fémur (figure 1).

Eventuellement, en fonction du type de fracture à traiter, le clou (2) est verrouillé au niveau de sa partie distale ou proximale. Par exemple, le clou (2) est verrouillé au moyen de vis (4) (figure 3).

Compte-tenu de la conception de l'ensemble prothétique selon l'invention, le mode opératoire peut être décrit comme suit :
- Ablation de l'implant initial (dans le cas de reprise).
- Alésage du canal au moyen d'alésoirs montés sur un guide centromédullaire.
- Mise en forme de la partie métaphysaire.
- Sélection de la taille de l'élément métaphysaire.
- Mise en place de l'élément métaphysaire au moyen du guide.
- Impaction de l'élément métaphysaire.
- Introduction du clou sur le guide.
- Verrouillage de l'ensemble élément métaphysaire (1) - clou (2).

Les avantages ressortent bien de la description, en particulier on souligne et on rappelle :
- la possibilité de solidariser le grand trochanter avec l'ensemble de l'implant éventuellement complété par une vis (5).
- d'autoriser l'enclouage centromédullaire.
- la possibilité de rendre extractible la partie diaphysaire de l'implant, si nécessaire, en enlevant simplement le clou.
- l'adaptation automatique de l'ensemble en fonction de l'évolution et du remodelage de l'os, au fur et à mesure de son veillissement, par glissement de l'élément métaphysaire par rapport au clou et mise en place éventuelle d'une vis au niveau du grand trochanter.

## Revendications

**-1-** Ensemble prothétique fémoral, caractérisé en ce qu'il comprend un élément (1) dont la forme et le profil en section sont déterminés pour permettre son impaction au niveau de la partie métaphysaire du fémur (F), ledit élément (1), qui présente directement ou de manière rapportée une tête d'articulation (3) coopérant avec la cavité cotyloïde, étant agencé pour l'introduction d'au moins un clou centromédullaire (2).

**-2-** Ensemble selon la revendication 1, caractérisé en ce que l'élément métaphysaire(1) présente un canal débouchant (1c) pour l'introduction du clou centromédullaire (2).

**-3-** Ensemble selon la revendication 1, caractérisé en ce que le clou (2) est fixé au niveau de la partie distale.

**-4-** Ensemble selon la revendication 1, caractérisé en ce que le clou (2) est fixé au niveau de la partie proximale .

**-5-** Ensemble selon la revendication 1, caractérisé en ce que le clou est déformable élastiquement.

**-6-** Ensemble selon la revendication 1, caractérisé en ce que le clou (2) est rectiligne.

**-7-** Ensemble selon la revendication 1, caractérisé en ce que le clou (2) est cintré.

**-8-** Ensemble selon la revendication 1, caractérisé en ce que l'élément métaphysaire (1) présente un col latéralisé (1b) recevant directement ou d'une manière rapportée, une tête sphérique d'articulation (3) coopérant avec la cavité cotyloïde.

**-9-** Ensemble selon la revendication 1, caractérisé en ce que le clou et la partie métaphysaire peuvent être verrouillés pour solidariser le grand trochanter.
